# EUROPEAN PATENT SPECIFICATION

(11) **EP 3 998 939 B1**
(45) Date of publication and mention of the grant of the patent: **31.12.2025**
(21) Application number: 20843503.2
(22) Date of filing: 17.07.2020
(51) Int. Cl.: A61B 5/02, A61B 5/0215

(54) **FUNCTIONAL IMPACT OF VASCULAR LESIONS**
FUNKTIONELLE AUSWIRKUNGEN VON VASKULÄREN LÄSIONEN
RÉPERCUSSIONS FONCTIONNELLES DE LÉSIONS VASCULAIRES

(30) Priority: 19.07.2019 US 201962875979 P
(43) Date of publication of application: 25.05.2022
(73) Proprietor: Cathworks Ltd., Kfar Saba 4465141 (IL)
(72) Inventor: LAVI, Ifat, 4069500 Moshav Mishmeret (IL); CORBETT, James Michael, San Juan Capistrano, CA 92675 (US); LAVI, Guy, 4069500 Moshav Mishmeret (IL); SEMO, Sarit, 4360705 RaAnana (IL)
(74) Representative: Liesegang, Eva
(86) International application number: PCT/US2020/042500
(87) International publication number: WO 2021/016071

(56) References cited:
- US-A1- 2015 250 395
- US-A1- 2016 157 802
- US-A1- 2016 247 279
- US-A1- 2018 243 033
- US-A1- 2018 243 033
- RABBAT MARK G. ET AL: "Interpreting results of coronary computed tomography angiography-derived fractional flow reserve in clinical practice", JOURNAL OF CARDIOVASCULAR COMPUTED TOMOGRAPHY, vol. 11, no. 5, 25 August 2017 (2017-08-25), AMSTERDAM, NL, pages 1 - 6, XP093057032, ISSN: 1934-5925, DOI: 10.1016/j.jcct.2017.06.002

## Description

### TECHNICAL FIELD

The present invention, in some embodiments thereof, relates to the field of vascular imaging and more particularly, to determination of vascular state using vascular images.

### BACKGROUND

Cardiac catheterization is a diagnostic test that allows a physician to evaluate the narrowing of the coronary arteries, based on an angiography and to determine the need for further treatment. Additional imaging procedures, such as intra-vascular ultrasound (IVUS) and fractional flow reserve (FFR), may be performed along with cardiac catheterization in some cases to obtain detailed images of the walls of the blood vessels. Following the diagnostic procedures, various treatment options will be considered. Treatment may include medication, coronary angioplasty (with or without coronary artery stenting), or coronary artery bypass surgery. Treatment is aimed at reducing or eliminating symptoms and reducing the risk of having a heart attack.

Fractional flow reserve (FFR) is a pressure management technique that is used to measure pressure gradient across a coronary stenosis to determine the ratio between the maximum achievable blood flow in a diseased coronary artery and the theoretical maximum flow in a normal coronary artery. Use of FFR information potentially improves PCI decision-making and outcomes. For example, the FAME trial (Tonino Pal, et al. Fractional Flow Reserve versus Angiography for Guiding Percutaneous Coronary Intervention. N Engl J Med. January 15, 2009; 360:213-224) randomized 1,005 patients to FFR-guided angiography with a≤. 80 FFR cutoff point vs. estimated visual assessment (angiography) alone as a basis to determine treatment (PCI or no PCI). Highlights of study findings include:
- 30% of patients indicated for stents with estimated visual assessment did not need to receive stents when evaluated with FFR.
- The number of stents used was greater by about one-third when the PCI decision was based on angiography alone versus FFR (2.7 vs. 1.9, P<0.001).
- One-year MACE (Major Adverse Cardiac Event) was higher in the visual angiography assessment arm compared to the FFR assessment arm (18.3% vs. 13.2%, P<0.02).
- Average cost-per-procedure for patients evaluated with FFR was reduced by 11.2% compared to angiography-only.

US 2016 / 157802 A1 discloses a method of evaluating risk associated with a condition of the vessel and providing an objective intervention recommendation based on the evaluated risk. The method includes obtaining physiologic measurements from a first instrument and a second instrument positioned within the vessel of the patient while the second instrument is moved longitudinally through the vessel from a first position to a second position, obtaining image data from an image of a vessel system, co-registering the physiologic measurements with the image data to produce co-registered physiologic measurements, and determining whether to perform a first surgical procedure or a second surgical procedure, wherein the determining is based on the co-registered physiologic measurements.

US 2018 / 0243033 A1 discloses a method including: receiving, in an electronic storage medium, a patient-specific representation of a portion of vasculature of the patient having a lesion at one or more points; receiving values for one or more metrics of interest associated with one or more locations in the vasculature of the patient; receiving one or more observed lumen measurements of the vasculature of the patient; determining the location of a diseased region in the vasculature of the patient using the received values for the one or more metrics of interest, wherein the determination of the location includes predicting or receiving one or more healthy lumen measurements of the vasculature of the patient; determining the extent of the diseased region ; and generating a visualization of the diseased region.

US 2015/0250395 A1 discloses a medical image processing apparatus including first specifier, second specifier, determiner and display controller. First specifier collates an ischemic region calculated from a blood vessel visualized into a three-dimensional image in a plurality of phases with a dominating region of the blood vessel, and specifies a culprit vessel in the ischemic region. Second specifier specifies a culprit stenosis in the culprit vessel based on a pressure index calculated from the blood vessel. Determiner determines a connection position to connect a bypass vessel that makes a detour around the culprit stenosis. Display controller displays the determined connection position on a display.

### SUMMARY

The current invention is defined in the appended claims. There is provided, in accordance with some embodiments of the present disclosure, a method of estimating a clinical state of a vascular portion, the method comprising: receiving a map of fractional flow reserve (FFR) assigning a multiplicity of FFR values to particular positions on each of a plurality of vascular segments of a vascular tree representing the vascular portion; and calculating an FFR impact score using the mapped FFR values, wherein an element of the FFR impact score discards the mapping of FFR values to the particular positions.

In some embodiments, the map of FFR continuously or near-continuously assigns FFR values to the particular positions.

In some embodiments, the map of FFR includes at least 5 FFR values for each of at least 4 vascular segments.

In some embodiments, the map of FFR represents contributions in reduction to flow capacity from an upstream position common to each of the particular positions.

In some embodiments, the vascular tree models a plurality of vascular extents of the vascular portion connected at branch points, and each vascular segment extends between two of the following: an origin of the vascular tree; a first vascular branch point; a second vascular branch point; an unconnected terminus of the vascular tree.

In some embodiments, the FFR impact score comprises a plurality of score elements, each of which discards the mapping of FFR values to the particular positions, but retains an association to a particular one of the plurality of vascular segments.

In some embodiments, the plurality of score elements comprise a total drop score for each of the plurality of vascular segments, representing a total drop in FFR along the vascular segment from a reference value representing an unoccluded vasculature.

In some embodiments, the method comprises assigning to each of the plurality of vascular segments a status as occluded or unoccluded, based on at least the total drop score for the vascular segment, counting the number of occluded vascular segments, and providing the sum as a multivessel score which is a score element of the FFR impact score.

In some embodiments, the plurality of score elements comprise a maximum drop score for each of the plurality of vascular segments, representing a maximum drop in FFR along the vascular segment along a defined portion of the vascular segment shorter than the vascular segment overall.

In some embodiments, the defined portion is a distance which encloses a blood volume with a range between about 10 mm³ and about 100 mm³.

In some embodiments, the defined portion is a distance which encloses a blood volume of about 40 mm3.

In some embodiments, the method comprises assigning to each of the plurality of vascular segments a status as occluded or unoccluded, based on at least the maximum drop score for the vascular segment, counting the number of occluded vascular segments, and providing the sum as a multivessel score which is a score element of the FFR impact score.

In some embodiments, the plurality of score elements comprise a diffused score for each of the plurality of vascular segments, representing a measure of how widely distributed along the vascular segment lesions contributing to the total drop in FFR are.

In some embodiments, calculating the vascular drop diffused score comprises determine the degree to which the maximum drop score differs from the total drop score.

In some embodiments, calculating the vascular drop diffused score comprises calculating a ratio of the total drop score and the maximum drop score.

In some embodiments, the plurality of score elements comprise a severity score, representing a weighted average of FFR in all locations in the vascular tree, weighted downward for locations at increasingly large distance from an origin of the vascular tree.

In some embodiments, the plurality of score elements comprises one or more score elements associated with a segment comprising the left main coronary artery up to a first branch point of the left main coronary artery represented within the vascular tree.

In some embodiments, the FFR impact score comprises a sorted-value chart score element which represents the FFR values of each of the plurality of vascular segments in a combined value-sorted order, such that values from different vascular segments are interleaved with each other.

In some embodiments, the method comprises displaying the sorted-value chart score element as a color-coded circle graph.

In some embodiments, the FFR impact score comprises a histogram chart score element which represents the FFR values of each of the plurality of vascular segments in a combined histogram, wherein the contribution of each FFR value to the histogram is weighted according to the magnitude of vascular volume within which the FFR value occurs.

In some embodiments, the FFR impact score comprises a score element describing a lesion length based on a distance over which an FFR value is continuously decreasing.

In some embodiments, the FFR impact score comprises a score element describing a lesion geometry as one or more of the following: including both a main vessel and a side branch; including a main vessel and at least two of its branches; occurring within an aorto-ostial vascular segment; occurring adjacent to a tortuous region of vasculature; occurring within a tortuous region of vasculature.

In some embodiments, the method comprises adjusting the FFR impact score, based on a revised map of FFR, revised according to one or more of the following: automatic virtual stenting; manual state selection; data measured after a stent implantation; data measured through a plurality of diagnostic procedures.

In some embodiments, the method comprises comparing the FFR impact score to a second FFR impact score calculated according to the method of claim 1, and estimating a rate of progression of vascular disease.

In some embodiments, the method comprises scheduling a further diagnostic procedure, based on the estimating.

In some embodiments, the method comprises planning a treatment procedure, based on the FFR impact score.

In some embodiments, the method comprises selecting between an OMT and PCI treatment, based on the FFR impact score.

In some embodiments, the method comprises selecting between a PCI and CABG treatment, based on the FFR impact score.

In some embodiments, the method comprises planning at least one of the number, location, and or type of stents to place, based on the FFR impact score.

In some embodiments, the method comprises planning at least one of the number and/or location, of CABG grafts to place, based on the FFR impact score.

There is provided, in accordance with some embodiments of the present disclosure, a method of estimating a clinical state of a vascular portion, the method comprising: receiving a map of fractional flow reserve (FFR) assigning a multiplicity of FFR values to particular positions on each of a plurality of vascular segments of a vascular tree representing the vascular portion; and calculating an FFR impact score using the mapped FFR values, wherein the FFR impact score comprises an element comparing a total drop in FFR along at least one of the vascular segments to a maximum drop in FFR along the vascular segment.

There is provided, in accordance with some embodiments of the present disclosure, a method of estimating a clinical state of a vascular portion, the method comprising: receiving a map of fractional flow reserve (FFR) assigning a multiplicity of FFR values to particular positions on each of a plurality of vascular segments of a vascular tree representing the vascular portion; and calculating an FFR impact score using the mapped FFR values, wherein the FFR impact score comprises a graph combining individual FFR values from a plurality of vascular segments into a display in which at least some of the individual FFR values are shown in graph positions which are non-adjacent to any other FFR value obtained from an adjacent vascular position.

There is provided, in accordance with some embodiments of the present disclosure, a system comprising a processor configured to perform the method described above,

Unless otherwise defined, all technical and/or scientific terms used herein have the same meaning as commonly understood by one of ordinary skill in the art to which the present disclosure pertains. Although methods and materials similar or equivalent to those described herein can be used in the practice or testing of embodiments of the present disclosure, exemplary methods and/or materials are described below. In case of conflict, the patent specification, including definitions, will control. In addition, the materials, methods, and examples are illustrative only and are not intended to be necessarily limiting.

As will be appreciated by one skilled in the art, aspects of the present disclosure may be embodied as a system, method or computer program product. Accordingly, aspects of the present disclosure may take the form of an entirely hardware embodiment, an entirely software embodiment (including firmware, resident software, microcode, etc.) or an embodiment combining software and hardware aspects that may all generally be referred to herein as a "circuit," "module" or "system" (*e.g*., a method may be implemented using "computer circuitry"). Furthermore, some embodiments of the present disclosure may take the form of a computer program product embodied in one or more computer readable medium(s) having computer readable program code embodied thereon. Implementation of the method and/or system of some embodiments of the present disclosure can involve performing and/or completing selected tasks manually, automatically, or a combination thereof. Moreover, according to actual instrumentation and equipment of some embodiments of the method and/or system of the present disclosure, several selected tasks could be implemented by hardware, by software or by firmware and/or by a combination thereof, *e.g*., using an operating system

For example, hardware for performing selected tasks according to some embodiments of the present disclosure could be implemented as a chip or a circuit. As software, selected tasks according to some embodiments of the present disclosure could be implemented as a plurality of software instructions executed by a computer using any suitable operating system In some embodiments of the present disclosure, one or more tasks performed in method and/or by system are performed by a data processor (also referred to herein as a "digital processor", in reference to data processors which operate using groups of digital bits), such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well. Any of these implementations are referred to herein more generally as instances of computer circuitry.

Any combination of one or more computer readable medium(s) may be utilized for some embodiments of the present disclosure. The computer readable medium may be a computer readable signal medium or a computer readable storage medium A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device. A computer readable storage medium may also contain or store information for use by such a program, for example, data structured in the way it is recorded by the computer readable storage medium so that a computer program can access it as, for example, one or more tables, lists, arrays, data trees, and/or another data structure. Herein a computer readable storage medium which records data in a form retrievable as groups of digital bits is also referred to as a digital memory. It should be understood that a computer readable storage medium, in some embodiments, is optionally also used as a computer writable storage medium, in the case of a computer readable storage medium which is not read-only in nature, and/or in a read-only state.

Herein, a data processor is said to be "configured" to perform data processing actions insofar as it is coupled to a computer readable memory to receive instructions and/or data therefrom, process them, and/or store processing results in the same or another computer readable storage memory. The processing performed (optionally on the data) is specified by the instructions. The act of processing may be referred to additionally or alternatively by one or more other terms; for example: comparing, estimating, determining, calculating, identifying, associating, storing, analyzing, selecting, and/or transforming. For example, m some embodiments, a digital processor receives instructions and data from a digital memory, processes the data according to the instructions, and/or stores processing results in the digital memory. In some embodiments, "providing" processing results comprises one or more of transmitting, storing and/or presenting processing results. Presenting optionally comprises showing on a display, indicating by sound, printing on a printout, or otherwise giving results in a form accessible to human sensory capabilities.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, *etc.,* or any suitable combination of the foregoing.

Computer program code for carrying out operations for some embodiments of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present disclosure may be described below with reference to flowchart illustrations and/or block diagrams of methods, apparatus (systems) and computer program products according to embodiments of the present disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

### BRIEF DESCRIPTION OF THE SEVERAL VIEWS OF THE DRAWINGS

Some embodiments of the present disclosure are herein described, by way of example only, with reference to the accompanying drawings. With specific reference now to the drawings in detail, it is stressed that the particulars shown are by way of example, and for purposes of illustrative discussion of embodiments of the present disclosure. In this regard, the description taken with the drawings makes apparent to those skilled in the art how embodiments of the present disclosure may be practiced.

In the drawings:
FIG. 1A-1B are schematic flowcharts of methods for generating an FFR impact score, according to some embodiments of the present disclosure;
FIGs. 2A-2C represent displayed results of a method of calculating FFR impact, according to some embodiments of the present disclosure;
FIGs. 3A-3D schematically represent FFR impact scores (in the form of circle charts constructed as already described for circle chart), in relation to disease treatment options, according to some embodiments of the present disclosure;
FIG. 4 represents displayed results of a method of calculating FFR impact, according to some embodiments of the present disclosure;
FIG. 5 illustrates an example of screen output provided by a system adapatable for calculation and display of a total FFR score, according to some embodiments of the present disclosure;
FIG. 6 schematically represents a system for calculation of FFR impact scores, according to some embodiments of the present disclosure;
FIG. 7 schematically represents data and processing instruction components of a system for calculation of FFR impact scores, according to some embodiments of the present disclosure; FIG. 8 is a schematic flowchart of calculation of FFR impact score components, according to some embodiments of the present disclosure;
FIG. 9 schematically illustrates a method of calculating a severity average score, according to some embodiments of the present disclosure; and
FIG. 10 is a schematic flowchart of a method of graphing received mapped FFR values, according to some embodiments of the present disclosure.

### DETAILED DESCRIPTION

A broad aspect of some embodiments of the present disclosure relates to metrics summarizing a functional status of a vascular tree to provide information useful as direct input to medical intervention decision making. In some embodiments, the summarizing metrics summarize a measure of fractional flow reserve (FFR).

As the term "FFR" is used herein, an FFR result as such represents a measured and/or estimated ratio of present blood flow (i.e., in units of volume per unit time) through a particular region of a potentially obstructed blood vessel, compared to ideal blood flow through the same section in an otherwise equivalent, but unobstructed blood vessel. As the abbreviation FFR (fractional flow reserve) implies, an FFR result provides an indication of what fraction of "reserve" flow (prevented by vascular obstruction) can in principle be restored to the blood vessel if it were somehow (e.g., by treatment) returned to its fully unobstructed state. The lower the FFR value, the more flow is "in reserve", in this sense-and the potentially more severe is the ischemia.

Defined this way, a "true" FFR result is an idealization to which actual FFR results are an approximation, constrained by real-world limitations. In particular, the notional "unobstructed blood vessel" must be supplied by a set of calculations and/or assumptions, since it is not actually available to compare to a diseased version of the same actual blood vessel. Practical methods of measuring and/or estimating FFR differ based in part on how real measurements are used to approximate the ideal. Herein the term FFR measurement, FFR estimate, or FFR value (including mapped FFR values, as discussed below) is intended to encompass the results of all such methods of measuring and/or estimating.

It should be understood that values which provide the functional information and estimated capacity for flow restoration of FFR but are not *per se* representations of fractions (*e.g.,* in virtual of being renormalized), are also included herein as FFR values.

It should be understood, furthermore, that this definition of FFR takes a superset of an earlier-developed, *in vivo* pressure-sensor based measurement also called FFR (and herein referred to as "pressure sensor-based FFR" to distinguish it from "FFR" as just defined). Pressure sensor-based FFR results represent a ratio of pressure downstream of a certain baseline position to pressure at that baseline position; wherein the two positions are close enough in location that they can be expected to also be approximately equal (*e.g.,* in a ratio of 0.8 to 1 or higher) in the pressure that *would* be measured there, in a healthy and unobstructed vessel. When the ratio is smaller than about 0.8, there is considered to be an obstruction between the two measurement positions that may be causing ischemia. Pressure is treated as a stand-in for flow, based on the well-known equations of flow which relate flow, pressure, and flow resistance.

It may be understood that a pressure sensor-based FFR measurement, to be interpreted as estimating "true" fractional flow reserve, relies on certain assumptions that tend to simplify the true situation: *e.g*., that the upstream site is itself in a position free of further significant upstream obstructions (the technique as actually practiced places the upstream sensor at the inlet position), and/or that any further downstream obstructions are also relatively free of obstruction. Pressure can drop anyway along an unobstructed blood vessel if the distance is long enough. Overall, pressure sensor-based FFR measurements tends to coincide best with FFR as above-defined when measuring from two nearby locations, separated by a single, relative focal lesion partially obstructing blood flow.

In contrast, image-based FFR is potentially capable of estimating fractional flow reserve continuously along whole blood vessel segments; and even among several different blood vessel segments of the same vascular tree. Image-based FFR is optionally calculated by another means, for example, by using computational flow dynamics (CFD) to obtain estimated measures of pressure along a blood vessel (with pressure being used to calculated FFR similarly to pressure-sensor based FFR). In another example image-based FFR is calculated by comparison of CPD-determined flow in stenotic and virtually revascularized models of the same vasculature.

Correspondingly, image-based FFR allows FFR values to be assigned, not just for single regions between two measurement points, but for a multiplicity of regions along whole blood vessel segments; optionally continuously along whole blood vessel segments, and optionally a plurality of such blood vessel segments.

Herein, the term "vascular tree" is used to mean a model of a vascular portion (which is part of some particular vascular anatomy of, *e.g.,* a patient), and a "vascular segment" is a portion of the vascular tree. More particularly, the vascular tree models a plurality of vascular extents connected to each other via branch points. Vascular segments can be defined as representing one or more such vascular extents, wherein each vascular segment extends between two of the following:
- an origin of the vascular tree (*e.g.,* the aortic root, in the case of a vascular tree modeling cardiac arterial vasculature)
- a first vascular branch point;
- a second vascular branch point;
- an unconnected terminal end of the vascular tree.

An aspect of some embodiments of the present disclosure relates to use of estimates of a single functional metric calculated for multiple locations in each of a plurality of connected vascular branches as an estimate of the overall impact of occlusive vascular disease on the connected vascular branches.

International Patent Publication No. WO2014/064702 describes the use of a variety of vascular measurements collected automatically from angiographic images to a provide inputs to a vascular state scoring tool (VSST). For example, the SYNTAX Score VSST. The SYNTAX Score is an angiographic tool used to characterize the coronary vasculature disease state and predict outcomes of coronary intervention based on anatomical complexity. SYNTAX Score grades the complexity of the coronary artery disease, which also allows for comparison between patients and for more effective communication between the doctors. This scoring calculation method has been recommended by professional societies of medical heart-care specialists as an integral part of the decision making process in complex cardiovascular cases. The SYNTAX Score questionnaire requires inputs about a plurality of different vascular metrics, for example: the location and size of lesions; including, for example: degree of occlusion (for example, a threshold occlusion of >50% is provided for in the scoring instructions), shape and length, presence of thrombus, and/or tortuosity of the blood vessel. Alternative examples of VSST approaches potentially include, for example, a "Functional SYNTAX Score" (integrating physiological measurements-for example, vascular flow capacity, vascular elasticity, vascular autoregulatory capacity, and/or another measure of vascular function-with a SYNTAX Score-like tool), or a "Clinical SYNTAX Score" (integrating clinical variables-for example, patient history, and/or systemic and/or organ-specific test results-with a SYNTAX Score-like tool). Examples also include the AHA classification of the coronary tree segments modified for the ARTS study, the Leaman score, the ACC/AHA lesions classification system, the total occlusion classification system, and/or the Duke and ICPS classification systems for bifurcation lesions.

Surprisingly, the inventors have come to a new understanding that a continuous or near-continuous map of FFR for a vasculature is potentially capable of supplanting multi-parameter VSSTs, by conversion of maps of FFR estimates into a score built atop that single parameter. This score (which may be, for example, scalar, vector, tabular, and/or graphical in nature) is referred to herein as an "FFR impact" score.

The conversion, in some embodiments, comprises emphasizing information about disease state itself, while de-emphasizing (removing) certain information contingent on particulars of a subject's vascular geometry and/or the completeness of the vascular model. In particular, conversion, starting from a map of FFR, extracts FFR values from the context of particular positions along vascular extents to produce a score which does not include this association. In some embodiments, the conversion uses a multiplicity of FFR values (*e.g*., at least 3, 5, 10, 20, 30 or more FFR values) from each of a plurality (*e.g.,* 2, 3, 4, 5, 6, 7, 8, 9, 10 or more) of vascular segments, each defined between a branch end, a root start, and/or a position of vascular branching (bifurcation or trifurcation, for example). In some embodiments, the multiplicity of particular positions is defined continuously or near-continuously according to the following senses of the terms:
Herein "near-continuous" means, e.g., at least 10, 20, 30 or more estimates per vascular segment between identified major branch nodes, and more specifically estimates using data obtained at a sufficient resolution to distinguish the length of vascular portions *through which* FFR is changing (reducing)-that is, defining not just two points that a lesion lies between, but also defining where the lesion begins and ends. Herein, "continuous" means that an FFR estimate is provided for each unit of representing resolution (*e.g*., each pixel) along the length of a blood vessel, and optionally calculated using a continuous function and/or a collection of adjoining interpolation functions, each of which is itself a continuous function. Both continuous and near-continuous representations (also referred to herein as "maps") of FFR are potentially capable of providing the bases of FFR impact score calculation. Herein the term "FFR map" or, equivalently, "mapped FFR" corresponds to a representation of FFR which maps FFR values to distinct particular positions along vascular segment extents in a continuous or near-continuous fashion.

Herein, vascular maps which *as such* represent non-FFR values (*e.g.,* another measure of vascular function and/or state) that are converted to individual FFR values in the course of calculating an FFR impact score are also considered as belonging to the type of "FFR maps", and the converted individual FFR values as belonging to the FFR map; *e.g.,* the conversion itself defines a portion of the mapping of FFR values to vascular locations.

One way of conceptualizing the relevance of FFR to disease complexity is to consider two related but separate aspects of vascular disease evaluation as part of treatment planning.

One of those aspects is *severity of disease.* Values of mapped FFR can be compiled into at least two different metrics of severity, one focal, and one global. The global measure asks simply "what is the total drop" in flow compared to the ideal healthy vessel. This is, for example, the value of FFR measured at the end of the vessel. The focal measure asks, in effect, "are there major lesions," which can be converted to the question "what is the worst single drop" in flow; for example, taken as maximum drop within some window. Severe cases of vascular disease are likely to include severe focal constrictions that make obvious contributions to the reduced flow state, and moreover are poised-as they occlude further-to contribute to dangerous acute ischemia. Another aspect of severity, in some embodiments of the invention, is calculation of *how much* of the vascular tree is subjected to lowering of FFR, and to what extent. This can be calculated, for example, as the relative area above the curve (below the maximum FFR value of 1) when all FFR values are removed from their mapped locations, and graphed in sorted order. The larger the relative area, the greater the FFR impact. This represents a summation- or "integration"-type use of FFR data to evaluate disease state.

The other aspect is *treatability of disease.* Part of the current accepted standard of care-for suitable patients-is the implantation of stents, which act to open and hold open focal regions of vascular stenosis. However, if the vascular stenosis is not mainly due to constructions in focal regions, vascular stents may not be able to provide sufficient recovery of flow. Use of mapped FFR can distinguish between two cases with the same total drop in a blood vessel, but where one drops continuously and/or over multiple steps, and the other drops at just one or two focal locations of major lesioning. In this case, the calculation may comprise, for example, obtaining a ratio of total FFR drop along a vessel to the maximum FFR drop across a given volume along that vessel (optionally suitably normalized and/or offset). When the raw or suitably normalized ratio is low, more of the FFR drop is happening at the position of maximum FFR drop, and the lesion is more focal. When the raw ratio is higher, there is potentially more distribution of stenotic sites along the blood vessel. In some embodiments, an operation other than arithmetical division is used; for example, a non-linear operation a look-up table, or a machine learning result jointly linking total and maximum FFR drops to a database of patient outcomes.

To a first approximation, in some embodiments, mapped FFR is converted into an FFR impact score by combining "derivative" (or "slope") type information indicative of the focality of stenotic lesions (the higher the magnitude of the derivative, the more focal), and "integral" ("summation") type information indicative of the overall severity of impacts on perfusion and ischemia. Each of these three types of information is optionally normalized against the other as part of normalization.

It may be noted, finally, that calculations that can be performed on mapped FFR to produce an FFR impact score have a correspondence to several of the types of parameters that a VSST such as SYNTAX Score concerns itself with. Focality determined from mapped FFR estimates can be understood as a stand-in for lesion length. Total FFR drop is a metric of occlusion. Integral of FFR drop is a metric for where lesions occur-the nearer to the carotid artery (in terms of distance along the blood vessels), the more tissue area affected by restricted perfusion. Finally, if these metrics are compiled for each of a plurality of vascular segments on different major branches of the vascular tree, a score state for the whole vascular tree can be determined.

Insofar as an FFR impact scores potentially "gets at" many of the same underlying issues of lesion complexity as SYNTAX Score seeks to capture, it may also potentially be found to have a similar value for guiding treatments and/or predicting treatment outcomes. At the same time, an FFR impact score has some potential advantages over scoring methods like SYNTAX Score. By relying primarily (optionally solely) on a single basic type of input data (FFR), FFR impact avoids the problem of determining how to weigh together several disparate inputs. Calculations using FFR avoid the heuristics of SYNTAX Score, which may not be reproducibly followed by all scorers. Even if scoring is automated, relying on a threshold conditions can create significant noise in a single score. For example, SYNTAX Score includes threshold-defined estimates (at least 50% occluded) suitable for human decision making, but potentially subject to scorer "noise", especially for values near the threshold cutoff. Since high-stakes decisions must be made for individual cases, this noise has real impacts on outcomes wherein there is no way available to "average out" noise error.

Additionally, although SYNTAX Score calculations *can be* automated, it was originally designed for calculation by humans. FFR impact scores use metrics which are inherently automated. One example of the difference this makes is that human-calculated scoring methods tend to try to "throw away data early" so that it doesn't have to be recorded or returned to. Yes/no and/or roughly graded decisions are easier for human scorers to deal with. Automated methods can retain any amount of intermediate result data to any suitable degree of precision, so that even if a simple score is required as an end result, that simplification can be in a later step, potentially the last one to generate the vascular state score itself.

Before explaining at least one embodiment of the present disclosure in detail, it is to be understood that the present disclosure is not necessarily limited in its application to the details of construction and the arrangement of the components and/or methods set forth in the following description and/or illustrated in the drawings. Features described in the current disclosure, including features of the invention, are capable of other embodiments or of being practiced or carried out in various ways.

### Method of Generating FFR Impact Score

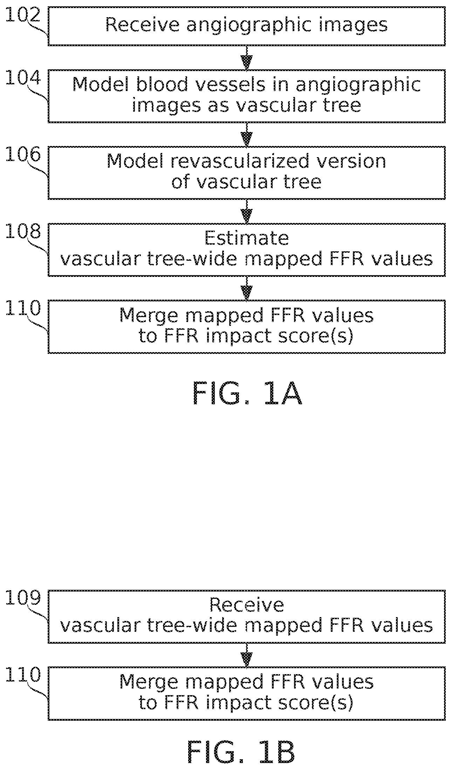

Reference is now made to *Figure 1A-1B**,* which are schematic flowcharts of methods for generating an FFR impact score, according to some embodiments of the present disclosure. At block 102, in some embodiments, angiographic images are received. In some embodiments, the angiographic images comprise a plurality of X-ray angiograms. In some embodiments, the angiographic images have another source, for example, CT, MRI, or another imaging modality. Herein, reference to "2-D imaging" in particular (and the "2-D images" it produces) relates to angiograms obtained by a projection imaging method which places a sensor at an imaging plane, and images radiant energy (*e.g.*, X-rays) which are received at the imaging plane after passing from a radiant energy source and through a body comprising tissue being imaged.

The angiographic images image a branching set of interconnected vascular segments comprising a portion of a vasculature. In some embodiments, the portion is a portion of a cardiac arterial vasculature.

At block 104, in some embodiments, imaged blood vessels shown in the angiographic images are converted into an angiographic model comprising a plurality of connected blood vessels; referred to herein as a "vascular tree". The longitudinal extents of segments of the vascular tree model correspond to longitudinal extents of corresponding segments of the imaged blood vessels. Moreover, cross-sectional geometries of the imaged blood vessels are represented in the model, optionally parameterized (*e.g*., as radius, diameter, and/or or area); optionally more fully described, for example, specified as a complete cross-sectional shape. Methods of generating such a model are described, for example, in International Patent Publication No. WO2014/111930, filed January 15, 2014.

At block 106, in some embodiments, regions of the vascular tree model which are determined to be stenotic (*e.g*., constricted and/or at least partially blocked oft) are "revascularized", in a modified model based on the vascular tree model. In the "revascularized" regions, vascular cross section information is modified, by a suitable method, to be like it would be in a stenosis-free state. In some embodiments, the revascularized cross-section is recovered by interpolating values between relatively unobstructed regions upstream and/or downstream of the stenosis itself. In some embodiments, other data, for example atlas information and/or consistency constraints are used to recover the revascularized diameters. Methods of generating revascularized vascular tree models are described, for example, in Patent Publication No. WO2014/111929, filed January 15, 2014.

At block 108, in some embodiments, vascular tree-wide FFR values are estimated. In some embodiments, the FFR value used is generated by comparing flow estimates through the original and the revascularized vascular tree models. FFR value calculation is described, for example, in International Patent Publication No. WO2014/111929, filed January 15, 2014. International Patent Publication No. WO2017/199245, filed May 16, 2017, describes color coding according to a cumulative vascular resistance along each vascular branch, and in particular display of vascular resistance corresponding to FFR calculated based on features of vascular image data.

This operations of blocks 102-108 are not necessarily the only approach capable of providing mapped FFR values. For example, computational flow dynamics (CFD) modeling of a suitably reconstructed blood vessel model could potentially be used to cumulatively determine a characteristic of the blood flow related to FFR along the extent of blood vessels; *e.g*., determine blood pressure at each point "as if' it had been directly measured as is performed in pressure-sensor based FFR. Methods of calculating FFR impact scores, in some embodiments, are applicable to such alternative sources of FFR data. Accordingly, the flowchart of *Figure 1B* simply begins at block 109 with receiving, vascular tree-wide FFR values.

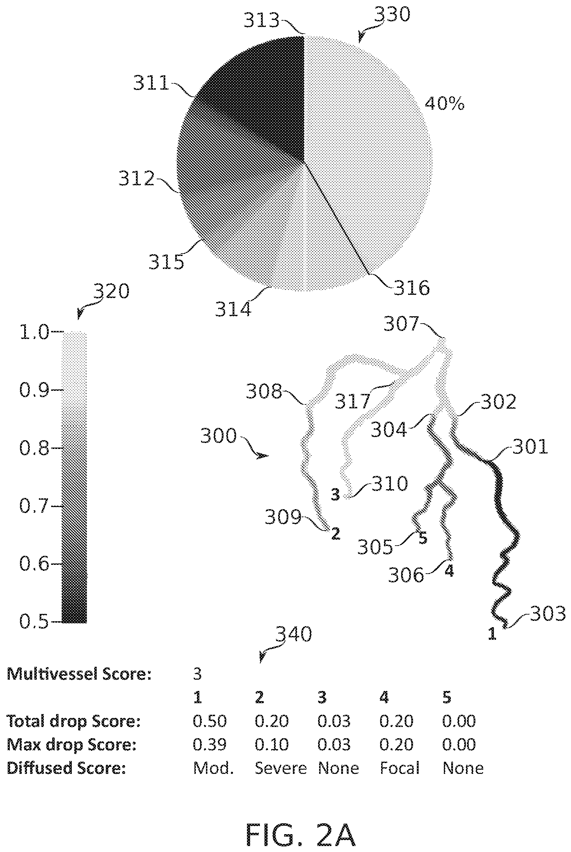

At block 110, in some embodiments (both *Figures 1A* and *1B*), estimated FFR values are merged to create one or more FFR impact scores, for example as explained in relation to *Figures 2A-2C**.*

### Image-based FFR

Reference is now made to *Figures 2A-2C**,* which represent displayed results of a method of calculating FFR impact, according to some embodiments of the present disclosure. Herein, the term "FFR impact score", in distinction from an FFR measurement as such, is used as a term for a score which describes how an overall distribution of FFR values within a vasculature impacts on the clinical state of a patient. Patients with different FFR impact scores are potentially suitable candidates for different treatment interventions.

Particular reference is first made to *Figure 2A**,* which illustrates a case comprising moderate to severe ischemic vascular disease in three of five annotated blood vessels.

The basis of an FFR impact score begins, in some embodiments, with individual FFR values measured and/or calculated locations along the vasculature, so as provide the FFR data of *Figures 1A* and/or *1B.*

Methods of image-based FFR calculations are described, for example, in International Patent Publication No. WO2014/111929, filed January 15, 2014. For example, in some embodiments, geometries of blood vessels are determined for large portions of a vascular tree based on vascular imaging (*e.g*., two-dimensional X-ray angiograms), evaluated for their capacity to be restored (this being what would restore the missing "reserve"), and the FFR value calculated based on this evaluation, for example by calculating how vascular resistance would change if the current vascular state was restored to a calculated geometry of an unobstructed state.

Image-based FFR's index range is optionally the same as for pressure sensor-based FFR index, with values ranging between O and 1. Optionally, 0.8 is retained as a cutoff value (in some embodiments, a different cutoff value, for example 0.75 or 0.85 is used), wherein a value above the cutoff value indicates that the lesion (if any) may-at least, in and of itself-be a non-ischemic lesion; and a value equal to or less than 0.8 indicates that the lesion may be an ischemic lesion.

In some embodiments, FFR values are assigned all along a 3-D or other model of the vasculature (for example as shown by vascular tree 300 of *Figure 2A*)*.* For purposes of display, in some embodiments, the 3-D model is optionally color coded according to the FFR estimate at each of its locations (in *Figure 2A**,* scale bar 320 shows a correspondence between FFR and gray level). Optionally, individual FFR values may be viewed; e.g., by using a cursor to select a particular location of the coronary vascular tree model.

### FFR Impact Scoring

With continuing reference to *Figures 2A-2C**,* a further analysis, in some embodiments, comprises converting mapped FFR (*e.g.,* as shown in the color-coded map of FFR along vascular extents) into a measure that can be readily understood by a physician in terms of FFR's impact on the clinical state of a vasculature, particularly its impact in relation to how effective one or more potential treatment options might be in restoring vascular function (that is, in restoring the missing "fractional flow reserve").

### Graphical FFR Impact Scoring

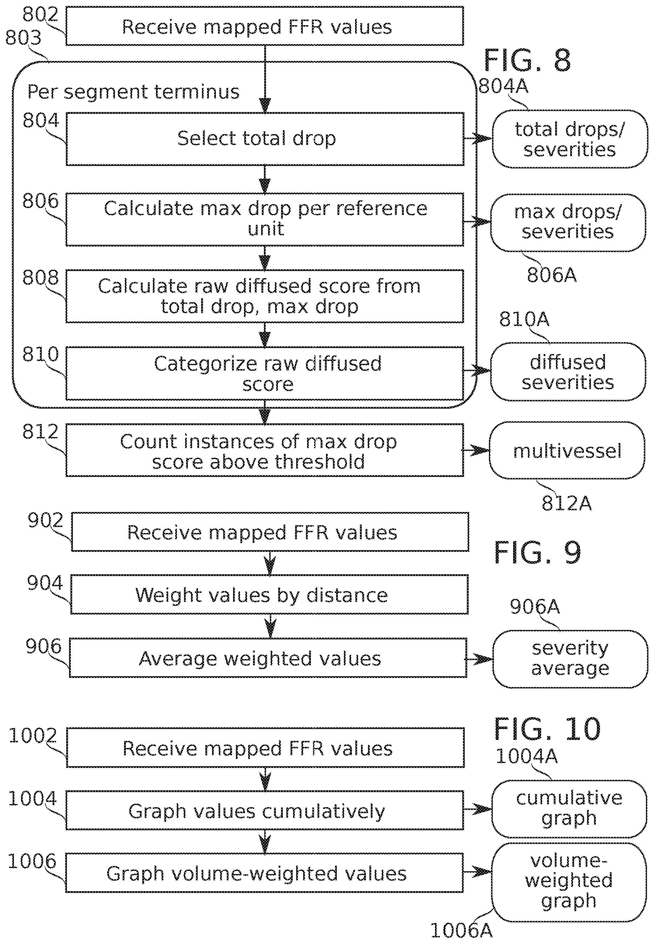

Brief reference is now made to *Figure 10**,* which is a schematic flowchart of a method of graphing received mapped FFR values, according to some embodiments of the present disclosure. At block 1002, in some embodiments, mapped FFR values are received. At block 1004, in some embodiments, the FFR values are converted into a cumulative graph, represented by cumulative graph 1004A. Circle graph 330 (*Figure 2A*) illustrates an example of a cumulative graph 1004A; displaying mapped FFR in a way which abstracts away vascular geometry to bring out patterns of vascular lesioning indicated by the FFR data itself. FFR values which in vascular tree 300 are shown distributed along blood vessels are instead sorted in descending order (values closest to 1 being the largest), and plotted as color-coded (grayscale-coded) radii in a clockwise direction around a circle, with equal angular distances being given to equal distances along the vascular extents. This combines data from all measured blood vessels (five terminal branches and their shared trunk segments) into a single vascular- tree wide display.

Circle graph 330 enables ready visual discrimination of numbers of lesion-created steps in FFR. Four significant post-lesion areas can be distinguished beginning at angles 314, 315, 312, and 311, corresponding to the four lesions located at vascular locations 302, 301, 308 and 304. There is also a minor lesion along the blood vessel terminating at vascular location 310; the corresponding FFR drop between angle 316 and 314 has been emphasized by drawing a line to divide the 40% region of no drop between angles 313 and 316 from the minor FFR drop between angles 316 and 314. In an example where some lesions have more similar characteristics, two or more steps would tend to be blended into a single step. Between these areas, steps in shading are separated by relatively short (focal lesion) or long (distributed lesion) transitions.

Numbers and sharpness of steps are "derivative related", in that they summarize information about, *e.g*., how many stents or would be needed to fully revascularize the vascular tree, and whether this revascularization can be achieved by focal corrections in vascular diameter.

Circle graph 330 also gives information about magnitudes of ischemic change (FFR grayscale values correspond to numbers shown on scale bar 320), as well as how much of the vasculature overall is affected (corresponding to "integral"-type information). The "40%" number shows how much vasculature is unaffected; while the angle that crosses the nominal ischemic line of 0.8 (angle 314) shows that almost half of the total vascular tree is experiencing ischemic flow.

As already mentioned, vascular lesions are somewhat blended together by this representation. For example, the values shown from angle 313 to angle 316 combine approximately the unique vascular extents extending between vascular location 307, and each of vascular locations 308, 317, 304, and 302. Furthermore, gradations in FFR are interlaced among blood vessels and spread out radially; *e.g*., values contributed by the extent between vascular locations 301 and 302 are spread out approximately between angle 314 and angle 311, intermingled with values contributed by extents between vascular locations 317 to 310, 308 to 309, and 304 to 305 and 306. The extent between vascular locations 301 and 303 (being the extent having a maximum drop in FFR) corresponds solely to the region between about angle 311 and angle 313.

These aspects of the visual pattern of circle graph 330 may be understood to at least generally correspond to disease severity, and in particular, disease severity as indexed by the treatment appropriate to it, for example as next discussed.

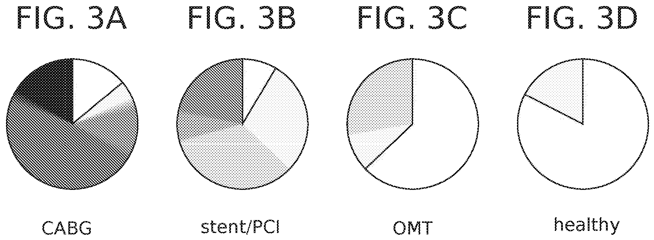

Reference is now made to *Figures 3A-3D**,* which schematically represent FFR impact scores (in the form of circle graphs constructed as already described for circle graph 330), in relation to disease treatment options, according to some embodiments of the present disclosure. Simply viewed qualitatively, it can be seen at a glance that the four cases given cover an ordered range of disease severity, from most severe in *Figure 3A**,* to most healthy in Figure 3D.

Conversion of these qualitative impressions to quantities is discussed after first briefly discussing characteristics of standard treatment options currently available.

In generally increasing order of disease severity to which they apply, commonly available treatment options for coronary artery disease currently may be divided into:

| | |
|---|---|
| Do nothing | The patient is healthy. |
| OMT | "Optimal medical therapy"-a relatively mild disease state may be managed by a suitable combination of non-surgical interventions such as drugs, diet, and behavioral and/or environmental changes. |
| Stent | Also known as percutaneous coronary intervention (PCI). One or more devices are inserted into the vasculature to create and/or maintain dilation. Stents may also be provided with eluting substances to prevent clotting, promote stability, and/or resist further accumulation of vascular lesion material. |
| CABG | Coronary artery bypass graft. Diseased vessels are entirely bypassed by use of grafts, *e.g*., using material obtained from other blood vessels of the same patient. While this is appropriate for the most advanced disease, it is also the intervention which carries with it the greatest risk to the patient. |

A typical criterion applied to distinguish between the three treatment options is to distinguish one- two- and three-artery disease as the markers for OMT, stent, and CABG, respectively. However, this rule of thumb potentially results in patients being miscategorised; for example, two-artery disease which is actually likely to be refractory to treatment by stent placement, or three-artery disease which is nevertheless amenable to treatment by stent placement. A similar potential for mis-categorizing exists between one and two vessel disease. Physicians are aware of this, and generally use multiple criteria in their decision making. However it is still occasionally unclear for borderline patients what treatment is appropriate. Existing numerical scoring methods such as SYNTAX score can help to resolve this, but are themselves subject to certain limitations as discussed herein.

A score which retains more of the nuance of the overall distribution of lesioning in the vasculature, yet abstracts away enough of it to allow direct comparison of patterns (and, eventually, results), provides a potential benefit to optimal decision making.

One aspect of the problem is in determining whether fixing just a few lesions (that is, a number within the practical limits of stenting procedures) will actually lead to a sufficient improvement in coronary blood flow.

### Examples of Numerical FFR Impact Scoring

Tabular summary 340 of *Figure 2A* shows results of a tabular approach to generating an FFR impact score. Reference is also made to *Figure 8**,* which is a schematic flowchart of calculation of FFR impact score components, according to some embodiments of the present disclosure.

At block 802, in some embodiments, mapped FFR values are received.

The score, in some embodiments, comprises a plurality of components. Some components are calculated per vessels, as indicated by the caption of enclosing block 803, enclosing blocks 804, 806, 808 and 810.

At block 804, in some embodiments, a "total drop score" is selected (represented as an output at block 804A). For each blood vessel marked in the modeled vascular tree, the maximum cumulative flow impairment is selected (resulting, in the case of *Figure 2A**,* in five values). As noted already, three blood vessels reach values at multivessel score threshold of 0.8 (*e.g.,* 1.0 - 0.20 = 0.8). Effectively, this measures the difference between the terminal end of each marked blood vessel, and a reference value representing an unoccluded vasculature position; such as FFR at an origin point of the vascular tree, or simply the defined maximum FFR of 1.

At block 806, in some embodiments, a "max drop score" (represented as an output at block 806A) is determined. The max drop score indicates the fraction of the maximum drop which is due to a nominal "single focal lesion". For purposes of calculation, the maximum size of a single focal lesion is taken to be, for example, 40 mm³, or another maximum size; for example a size from within a range of values between about 10 mm³ and 100 mm³.

Block 810A, in some embodiments, represents the output of a diffused severity of lesioning (diffused severity score). As the max drop score becomes increasingly smaller than the total drop score, it is an indication that the overall vascular disease is increasingly diffuse along the blood vessel described. At block 808, in some embodiments, a raw diffused score is calculated as the ratio between the total drop score and the max drop score, optionally normalized (*e.g*., by subtracting 1) so that a score of 0 indicates "maximally focal" lesioning, and higher values indicate more diffuse lesioning. At block 810, in some embodiments, the diffuseness of the lesioning is optionally given a category ranking, *e.g*., None (no disease), Focal (diffused score less than, for example, 0.1), Moderate (diffused score up to, for example, 0.5), and Severe (diffused score larger than 0.5). In some embodiments, diffuseness is scored by another method, for example, a look-up table, a non-linear function, and/or a machine learning result jointly linking total and maximum FFR drops to a database of patient outcomes.

Block 812A, in some embodiments, represents a "multivessel score" output. At block 812, in some embodiments, the multivessel score is calculated.

In some embodiments, the multivessel score simply counts how many blood vessels have ischemic-level FFR values somewhere along their length (under a threshold criterion of, *e.g.,* 0.8, or optionally a higher values such as 0.9). In the example shown, the multivessel score is 3. It should be noted that a shared lesion-*e.g*., the lesion shared by vessels 4 (terminating at vascular location 306) and 5 (terminating at vascular location 305)-is counted only once; and assigned, for purposes of scoring, to one of the two branches (vessel 4, in this case).

In some embodiments, multivessel score 812A is based (additionally or alternatively) on a measure like the "max drop score" 806A, and includes vessels based on reaching at least a certain amount of drop in FFR values within a given volume (for example, within 403 mm, or another volume, for example, 20 mm³, 30 m³, 50 mm³ or 100 mm³). In some embodiments the range is selected from within a range of values between about 10 mm³ and 100 mm³. This range includes values which potentially allow a distinction between lesions focal enough to be good candidates for PCI treatments, and lesions which are more diffuse and potentially less suitable for PCT treatments.

In the example of *Figure 2A*: two vessels have no unique lesion, one has a "maximally focal lesion". Blood vessel 1 (terminating at vascular location 303) has two lesions, with the more severe of them accounting for over half the total drop. Blood vessel 2 appears to have only one distinct drop, but it accounts for only about half the total drop, indicating that disease diffusion in this blood vessel is "severe". These factors, considered together-and especially in view of the finding of severely diffuse disease in one of the vessels, even though it is not the most severely decreased in total FFR drop-indicate, in some embodiments, that CABG may be a preferred treatment for this patient, other risk factors permitting.

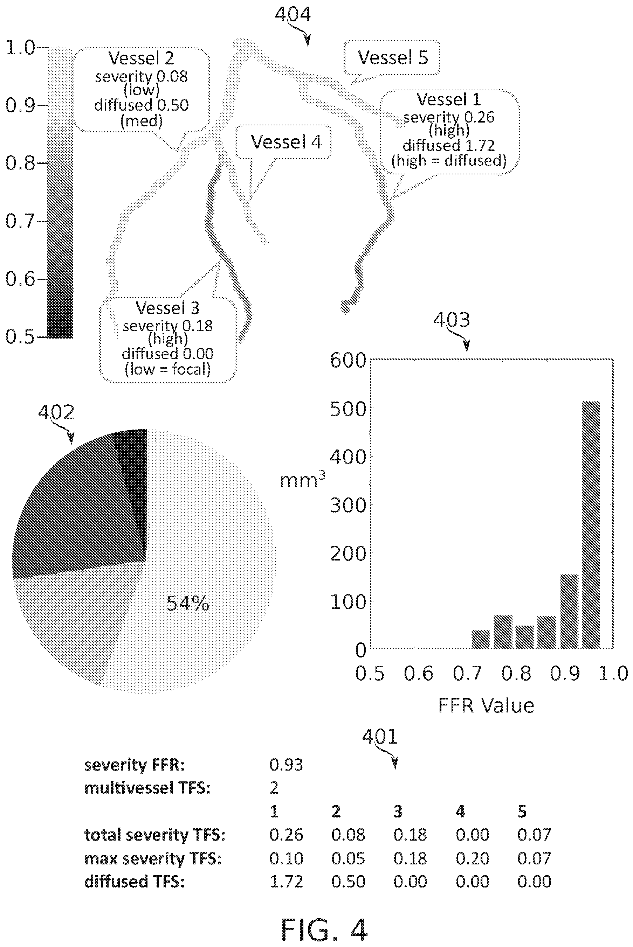

Other examples of FFR Impact score components are described herein in relation to *Figure 4**.*

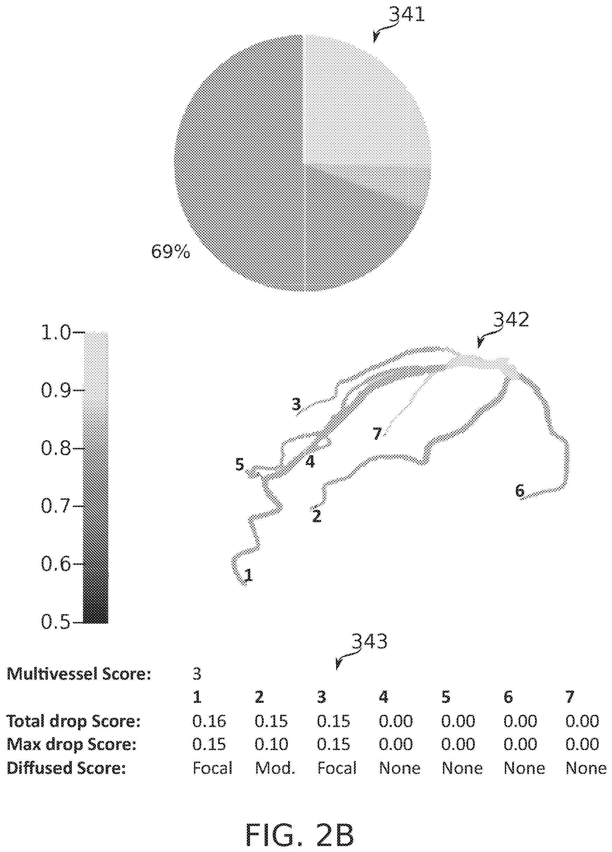

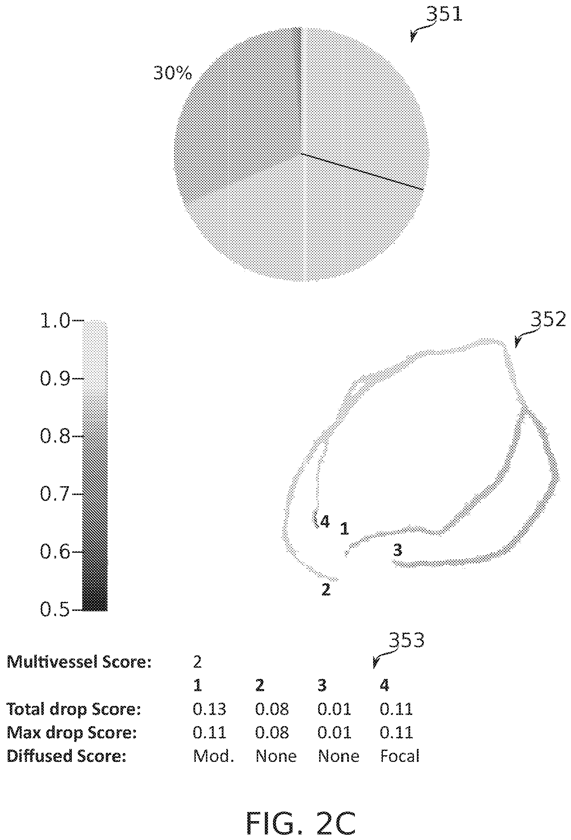

*Figures 2B* and 2C provide examples of different vascular states (assessed by FFR) and their associated FFR impact scores.

In *Figure 2B**,* (vascular tree 342, circle graph 341, and tabular summary 343) the largest portion of circle graph 341 (at 69%) has an FFR value well below 1, but still hovering above the pressure-sensor based FFR cutoff value of 0.80. Even so, this is a "three vessel disease" under stricter FFR criteria (multivessel score is 3, using an FFR criterion of 0.9 as the cutoff) in the sense of having occlusions in three or more vessels. Accordingly, the severity of the disease appears to be better suited to stenting (since all lesions are focal or moderate in diffusion). An option to continue with OMT (since all lesions are above the 0.80 cutoff) may be available, depending on accompanying risk factors counter-indicating PCI.

In the example of *Figure* 2C (vascular tree 352, circle graph 351, and tabular summary 353), all vessels are affected to some degree, but only two pass the total drop FFR cutoff value of 0.9, and the level of diffusion is focal, or moderate at worst. This subject, accordingly, is in some embodiments preferably placed on OMT, rather than receiving PCI which would otherwise be the baseline treatment selected for a two-vessel disease state.

It should be understood that there is no particular limitation to using a circle graph to chart the values of, *e.g.,* circle graph 341. In some embodiments, the graph is a Cartesian graph of values (*e.g*., X-axis as FFR value count and Y-axis distance as FFR value), a polar plot of values (*e.g*., angular axis as FFR value count and radius length as FFR value), or another graphing method.

### Additional Examples of Numerical FFR Impact Scoring

Reference is now made to *Figure 4**,* which represents displayed results of a method of calculating FFR impact, according to some embodiments of the present disclosure.

Displayed again are a vascular tree 404, a circle graph 402, and a tabular summary 401, which correspond in general to vascular tree 300, circle graph 320, and tabular summary 340 of Figure 2A. Labels of vascular tree 404 add severity and diffused result values (together with category evaluations) corresponding to those also shown in tabular summary 401.

In this example, "TFS" is used as an abbreviation for "Total FFR Score", used equivalently to the term "FFR impact score".

The "total severity TFS", "max severity TFS" and "diffused TFS", and "multivessel TFS" scores of *Figure 4* correspond to the "total drop", "max drop" and "diffused" scores of *Figure 2A**.*

In *Figure 4**,* a "severity FFR" score for the overall vascular tree is shown, which calculates mean FFR value for all locations in the tree, weighted downward as a function of increasing distance of each location from the ostium. A value of 1 is fully un-occluded. Weighting downward as a function of distance from the ostium allows greater importance to be given to lesions nearer to the root of the vascular tree, where their influence is correspondingly greater.

Reference is now made to *Figure 9*, which schematically illustrates a method of calculating a severity average score, according to some embodiments of the present disclosure. At block 902, in some embodiments, mapped FFR values are received. At block 904,

m some embodiments, the values are weighted by distance (downward with increasing distance). At block 906, in some embodiments, the severity score is calculated by averaging the FFR values according to their weight. Block 906A represents the severity score produced (as a weighted average).

Also shown in *Figure 4* is a bar graph 403 showing FFR per unit volume. Essentially, the length of the bars is weighted by volume, and not just distance along a vessel centerline. In this manner, vascular regions which are more downstream (in narrower vessel portions) are again given a somewhat lower weight. This has the potential advantage of corresponding to how much area is finally perfused by blood flowing through a certain portion of the blood vessel, and therefore the size of an area which is affected directly by ischemic flow in that portion.

Continued reference is made to *Figure 10**.* At block 1002, mapped FFR values are received. At block 1006, the mapped FFR values are graphed as volume-weighted values, using the vascular volumes associated with each mapped FFR value in the given vascular locations. Block 1006A represents a volume-weighted graph produced at block 1006.

In some embodiments, a subscore based on FFR is provided for "left main narrowing", wherein narrowing within the left main coronary artery (through which blood supply to the left side of the heart passes) is particularly singled out for attention, *e.g.*, by a metric such as the "total drop" or "max drop" calculation made for individual vascular branches.

In some embodiments, patterns in mapped FFR values are associated with particular additional features, which are optionally indicated in the FFR impact score. For example:

### Length of lesion

The length of a vascular region through which mapped FFR continuously decreases significantly. For purposes of this metric, significantly means, *e.g*., by more than a given threshold overall, for example, about 0.1. Continuously means, *e.g*., with a certain minimum slope amplitude, for example, at least 0.1 per 10 mm³, 20 mm³, 30 mm³, or 40 mm³, without interruptions; or optionally with interruptions only shorter/smaller than a certain threshold vascular distance or volume (*e.g*., less than 10 mm³, 20 mm³, 30 mm³, or 40 mm/\3).

### Main vessel + Side branch Lesion

A vascular region through which mapped FFR decreases significantly (*e.g*., by more than a given threshold, for example, about 0.1, optionally normalized to a particular vascular length and/or volume such as 40 mm³, or another volume; for example a volume from within a range of values between about 10 mm³ and 100 mm³) that extends from a main vessel past a vascular branch point into a branch vessel.

### Bifurcation / Trifurcation Lesion

A vascular region through which mapped FFR decreases significantly (*e.g*., by more than a given threshold, for example, about 0.1, optionally normalized to a particular vascular length and/or volume such as 40 mm³, or another volume; for example a volume from within a range of values between about 10 mm³ and 100 mm³) that extends through three or more vascular segments (*e.g.*, main vessel and two side vessels past a vascular bifurcation).

### Aorto-ostial lesion

The aorta and/or aortal ostium itself includes a region of significantly decreasing mapped FFR, *e.g*., decreasing more than a given threshold; for example, about 0.1, optionally normalized to a particular vascular length and/or volume such as 40 mm³, or another volume; for example a volume from within a range of values between about 10 mm³ and 100 mm³.

### Tortuosity

One measure of tortuosity is that a vascular segment shows excessive winding, such that there is a high ratio of distance along a curving vessel segment to a minimum distance between two endpoints of the vessel segment. For example, a tortuous vessel has a ratio of 1.3 or more. In some embodiments, an FFR impact score indicates the presence of tortuosity associated with (*e.g.*, beginning with, ending with, and/or or passing through) a vascular region through which mapped FFR decreases significantly. Significantly, in some embodiments, means by more than a given threshold, for example, about 0.1. Optionally, the decrease is normalized to a particular vascular length and/or volume such as 40 mm³, or another volume; for example a volume from within a range of values between about 10 mm³ and 100 mm³.

### Stent count/stent effectiveness estimate

By, for example, a suitable combination of the FFR impact score features just described and/or described in relation to *Figures 2A-2C**,* a stent count estimate can be created. For example, lesion lengths and complexities can be evaluated as suitable for numbers of stent implantations, which would restore flow function to a certain degree of improvement (*e.g*., to FFR of .85, .90, 0.95, or 1.0). Optionally or alternatively, the maximum number of stents is constrained based, *e.g.,* on an estimate of procedure risk and/or practicality, and the stent count estimate (*e.g*., potentially limited to a maximum value by risk) produced along with an estimate of the degree of improvement which could result.

### CABG count/CABG effectiveness estimate

By, for example, a suitable combination of the FFR impact score features just described and/or described in relation to *Figures 2A-2C**,* a graft count estimate can be created. For example, lesion lengths and complexities can be evaluated as suitable for numbers of grafts, which would restore flow function to a certain degree of improvement (*e.g*., to FFR of .85, .90, 0.95, or 1.0). Optionally or alternatively, the maximum number of grafts is constrained based, *e.g.,* on an estimate of procedure risk and/or practicality, and the graft count estimate (*e.g.,* potentially limited to a maximum value by risk) produced along with an estimate of the degree of improvement which could result.

### Revisions of FFR Impact Scoring

In some embodiments, a system configured to calculated FFR impact scores is also configured to recalculated FFR impact scores, using data describing measured and/or simulated changes in vascular state; for example: post-stenting angiographic images, and/or changes of the vascular tree model which simulate changes in disease state as a result, *e.g.,* of treatment and/or disease progression. Examples include:
An FFR impact score (*e.g.*, any the numeric, graphical, and/or tabular data shown in one of Figures 2A-2C and/or 4) is updated based on one more of:
- **Automatic virtual stenting:** vascular diameters at proximal and/or distal vascular positions are used as references for what constitutes the anticipated revascularized ("restored healthy") diameter after virtual stenting. The revascularized diameter is used to adjust the vascular tree model used to produce new FFR data; and the FFR impact score is recalculated accordingly. In some embodiments, options are displayed according a suitably weighted blend of the criteria of providing maximal improvements, and providing minimal intervention.
- **Manual stent selection:** According, *e.g*., provided stent parameters of location, length and diameter, the vascular tree model is adjusted to produce adjusted FFR data, and the FFR impact score is recalculated accordingly. Optionally, stent parameters available are constrained by known and/or available stent(s), for example, stents available in current inventory.
- **Post-PCI Data:** Post-stenting angiography images are used to adjust the vascular tree model. Adjusted FFR data is produced, and the FFR impact score is recalculated accordingly.
   1. **Follow up:** Angiographic image data acquired from a patient over the course of a plurality of diagnostic procedures (for example, performed over a period of several weeks, months or years), is converted into a plurality of perioddependent vascular tree models. Adjusted FFR data is produced for each model, and the FFR impact score is recalculated accordingly, resulting in outputs which describe changes in FFR impact score over the period spanned by image monitoring. In some embodiments, a comparison of two or more FFR impact scores from FFR data obtained over the course of the plurality of diagnostic procedures is used to estimate a rate of progression of vascular disease. In some embodiments, the rate of progression of vascular disease is used to schedule a further diagnostic procedure. Optionally, the comparison is optionally performed for FFR impact scores calculated taking into account only vascular segments scored in common for all FFR impact scores being calculated.

### Example of Implementing System Components

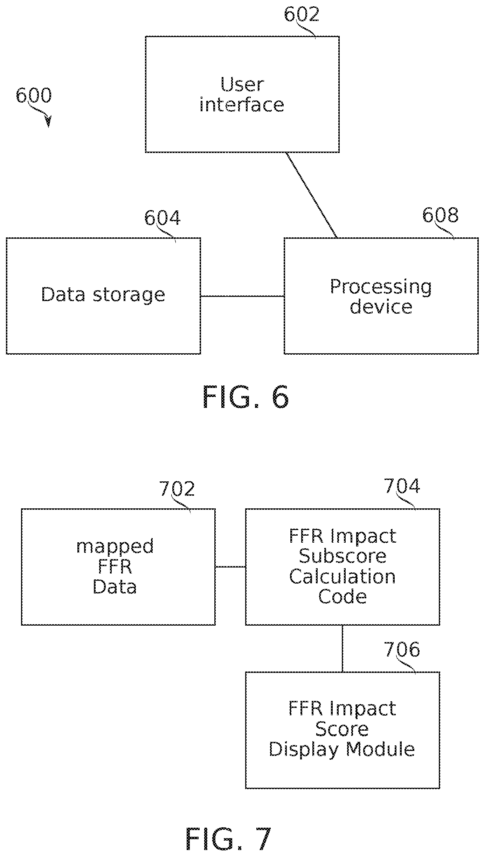

Reference is now made to *Figure 6**,* which schematically represents a system 600 for calculation of FFR impact scores, according to some embodiments of the present disclosure.

Data storage device 604, in some embodiments, stores FFR data (for example, in any of the forms described herein as a computer readable medium and/or memory. Optionally, data storage device 604 is used to store images used in the calculation of FFR, for example according to the method outlined in *Figure 1A**.*

Processing device 608, in some embodiments, carries out processing which converts FFR data into FFR impact scores, for example, according to any one or more of the calculations used to calculate FFR impact score components described herein, *e.g.,* in relation to *Figure 2A-2C* and/or *4*.

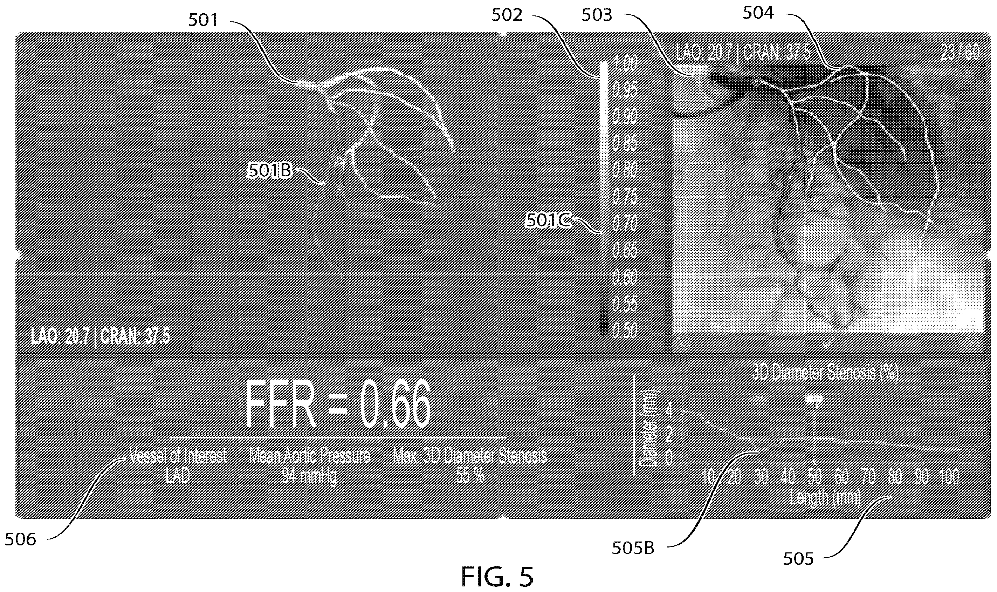

User interface 602, in some embodiments, accepts user input (for example to select and/or define data for calculation and/or provide commands to initiated and/or configure processing). In some embodiments, user interface 602 also displays FFR impact score information; for example in a format such as shown and/or described in relation to Figures 2A-4, herein. Optionally, the system of *Figure 6* is also a system for angiographic image analysis more generally; for example as described in relation to *Figure 5**.*

Reference is now made to *Figure 7*, which schematically represents data and processing instruction components of a system 600 for calculation of FFR impact scores, according to some embodiments of the present disclosure.

The mapped FFR data 702 comprises FFR data calculated, for example, as described in relation to *Figures 1A-1B**,* and stored in data storage 604.

The processing instruction components, in some embodiments, comprise computer code, for example stored by storage device 604 and processed by processing device 608. A portion of the processing instruction components comprises FFR impact subscore calculation code 704, which performs, in some embodiments, one or more of the subscore calculations described, for example, in relation to *Figures 2A-2C* and/or *4*. In some embodiments, the processing instruction components also include and FFR impact score display module, configured, for example, to convert the subscore calculations of impact subscore calculation code 704, into displays; for example, displays described in relation to *Figures 2A-4**.*

Reference is now made to *Figure 5**,* which illustrates an example of screen output provided by a system 600 adapatable for calculation and display of a total FFR score, according to some embodiments of the present disclosure.

In some embodiments, the system 600 is a non-invasive image-based software device that provides physicians with a quantitative analysis of functional significance of a coronary lesion, similar to invasive FFR, and a qualitative three-dimensional model of the demonstrated coronary arteries, during routine PCI procedure. For example, summary block 506, shows an FFR of 0.66 for a selected flow-restricted vessel 501B of a 3-D coronary artery model 501; the flow-restricted vessel 501B corresponding in color to the shade of the color bar 502 at 501C. Also shown is a centerline vascular tree 504, corresponding to 3-D coronary artery model 501, and superimposed on X-ray angiogram 503. The diameter of flow-restricted vessel 501B is graphed along graph 505, including a dip at location 505B corresponding to the location of flow restriction that is the primary contribution to lowering the FFR of flow-restricted vessel 501B from the fully healthy value of 1.00 to its indicated value of 0.66.

The system 600, in some embodiments, performs processing and calculations based on angiography images and hemodynamics information acquired during coronary catheterization procedures.

Optionally, system 600 does not require use of additional invasive devices or vasodilation treatment. Features and/or potential advantages of system 600 related to FFR-guided PCI decision-making include, in some embodiments:
- Delivery of intra procedural imaging, computation, and 3D multi-vessel modelling to quantify stenosis, optimize PCI decisions, and confirm end-of-case revascularization.
- Objective FFR measurement without the risks associated with invasive FFR wires including additional interventions and pharmacologic administration.
- Uses existing cath-lab imaging and angiography equipment - compatible with major angiography systems.
- Eliminates direct and indirect FFR wire costs, without introducing additional SKUs or device-related procedure costs.

Reconstructed 3-D models provided by the system 600 describe the shape and volume of the vessels, serving as a basis for blood flow analysis, together with hemodynamic parameters. Based on the blood flow analysis, FFR can be calculated for the inspected vessel. In some embodiments, blood flow calculations are based on a lumped model of the arteries, enabling short processing time for delivery of FFR results to the physician during the PCI procedure. After calculations, the system 600 presents a 3-D simulation of the coronary tree and the calculated FFR values for the vessel of interest as shown in *Figure 5**.*

In some embodiments, a total FFR score, for example as described in relation to *Figures 1A-4*, is calculated based on information provided by a suitably configured system 600; and in particular on FFR calculations for individual vessels and/or locations along individual vessels.

### General

As used herein with reference to quantity or value, the term "about" means "within ±10% of".

The terms "comprises", "comprising", "includes", "including", "having" and their conjugates mean: "including but not limited to".

The term "consisting of means: "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example" or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the present disclosure may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, embodiments may be presented with reference to a range format. It should be understood that the description in range format is merely for convenience and brevity and should not be construed as an inflexible limitation on the scope of descriptions of the present disclosure. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Although descriptions of the present disclosure are provided in conjunction with specific embodiments, it is evident that many alternatives, modifications and variations will be apparent to those skilled in the art. It is appreciated that certain features which are, for clarity, described in the present disclosure in the context of separate embodiments, may also be provided in combination in a single embodiment. Conversely, various features, which are, for brevity, described in the context of a single embodiment, may also be provided separately or in any suitable subcombination or as suitable in any other described embodiment of the present disclosure. Certain features described in the context of various embodiments are not to be considered essential features of those embodiments, unless the embodiment is inoperative without those elements.

## Claims

1. A method of estimating a clinical state of a vascular portion performed by a processing device (608), the method comprising:
receiving a map of fractional flow reserve (FFR) assigning a multiplicity of FFR values to particular positions on each of a plurality of vascular segments of a vascular tree representing the vascular portion;
calculating an FFR impact score using the mapped FFR values, the FFR impact score being indicative of an overall impact of occlusive vascular disease on the vascular tree;
wherein the FFR impact score comprises a cumulative graph, and
wherein the calculating the FFR impact score comprises:
removing the FFR values from their mapped locations; and
graphing the FFR values in sorted order,
wherein the graphing the FFR values further comprises:
converting the FFR values into the cumulative graph.

2. The method of claim 1, wherein the map of FFR continuously or near-continuously assigns FFR values to the particular positions.

3. The method of any one of claims 1-2, wherein the map of FFR represents contributions in reduction to flow capacity from an upstream position common to each of the particular positions.

4. The method of any one of claims 1-3, wherein calculating the FFR impact score comprises calculating a plurality of score elements, each of which comprises removing the mapping of FFR values from its mapped location, but retains an association to a particular one of the plurality of vascular segments.

5. The method of claim 4, wherein the plurality of score elements comprises a total drop score for each of the plurality of vascular segments, representing a total drop in FFR along the vascular segment from a reference value representing an unoccluded vasculature.

6. The method of any one of claims 4-5, further comprising assigning to each of the plurality of vascular segments a status as occluded or unoccluded, based on at least the total drop score for the vascular segment, counting a number of occluded vascular segments, and providing the sum as a multivessel score which is a score element of the FFR impact score.

7. The method of any one of claims 4-6, wherein the plurality of score elements comprises a maximum drop score for each of the plurality of vascular segments, representing a maximum drop in FFR along the vascular segment along a defined portion of the vascular segment shorter than the vascular segment overall.

8. The method of claim 7, further comprising assigning to each of the plurality of vascular segments a status as occluded or unoccluded, based on at least the maximum drop score for the vascular segment, counting the number of occluded vascular segments, and providing the sum as a multivessel score which is a score element of the FFR impact score.

9. The method of any one of claims 4-8, wherein the plurality of score elements comprise a diffused score for each of the plurality of vascular segments, representing a measure of how widely distributed along vascular segment lesions contributing to the total drop in FFR are.

10. The method of claim 9, wherein calculating the vascular drop diffused score comprises:
determining a degree to which the maximum drop score differs from the total drop score; or
calculating a ratio of the total drop score and the maximum drop score.

11. The method of any one of claims 4-10, wherein the plurality of score elements comprises:
a severity score, representing a weighted average of FFR in all locations in the vascular tree, weighted downward for locations at increasingly large distance from an origin of the vascular tree; or
one or more score elements associated with a segment comprising a left main coronary artery up to a first branch point of the left main coronary artery represented within the vascular tree.

12. The method of any one of claims 1-11, wherein the FFR impact score comprises:
a sorted-value chart score element which represents the FFR values of each of the plurality of vascular segments in a combined value-sorted order, such that values from different vascular segments are interleaved with each other;
a histogram chart score element which represents the FFR values of each of the plurality of vascular segments in a combined histogram, wherein a contribution of each FFR value to the histogram is weighted according to a magnitude of vascular volume within which the FFR value occurs; or
a score element describing a lesion length based on a distance over which an FFR value is continuously decreasing.

13. The method of claim 12, further comprising displaying the sorted-value chart score element as a color-coded circle graph.

14. The method of any one of claims 1-13, wherein the FFR impact score comprises a score element describing a lesion geometry as one or more of the following:
including both a main vessel and a side branch;
including a main vessel and at least two of its branches;
occurring within an aorto-ostial vascular segment;
occurring adjacent to a tortuous region of vasculature; or
occurring within a tortuous region of vasculature.

15. The method of any one of claims 1-14, further comprising adjusting the FFR impact score, based on a revised map of FFR, revised according to one or more of the following:
automatic virtual stenting;
manual state selection;
data measured after a stent implantation; or
data measured through a plurality of diagnostic procedures.

16. The method of any one of claims 1-15, further comprising comparing the FFR impact score to a second FFR impact score calculated according to the method of claim 1, and estimating a rate of progression of vascular disease.

17. The method of claim 16, further comprising at least one of:
scheduling a further diagnostic procedure, based on the estimating;
planning a treatment procedure, based on the FFR impact score;
selecting between an OMT and a PCI treatment, based on the FFR impact score;
selecting between a PCI and a CABG treatment, based on the FFR impact score;
comprising planning at least one of a number, location, and or type of stents to place, based on the FFR impact score; or
comprising planning at least one of a number and/or location, of CABG grafts to place, based on the FFR impact score.

18. An apparatus (600) for estimating a clinical state of a vascular portion, the apparatus comprising:
a data storage device (604) storing a data structure that assigns a multiplicity of fractional flow reserve (FFR) values to particular positions on each of a plurality of vascular segments of a vascular tree representing the vascular portion; and
a processing device (608) communicatively coupled to the data storage device and configured to:
calculate an FFR impact score using the assigned FFR values, the FFR impact scores being indicative of an overall impact of occlusive vascular disease on the vascular tree,
wherein the FFR impact score comprises a cumulative graph,
and wherein the calculating the FFR impact score comprises:
removing the FFR values from their mapped locations; and
graphing the FFR values in sorted order,
wherein the graphing the FFR values further comprises:
converting the FFR values into the cumulative graph, and
cause a user interface (602) to display the FFR impact score.

19. The apparatus of claim 18, wherein calculating the FFR impact score comprises calculating a plurality of score elements, each of which comprises removing the assignment of FFR values from its mapped location, but retains an association to a particular one of the plurality of vascular segments.

20. The apparatus of claim 19, wherein the plurality of score elements comprise a total drop score for each of the plurality of vascular segments, representing a total drop in FFR along the vascular segment from a reference value representing an unoccluded vasculature.

21. The apparatus of any one of claims 19 or 20, wherein the processing device is configured to:
assign to each of the plurality of vascular segments a status as occluded or unoccluded, based on at least the total drop score for the vascular segment;
count a number of occluded vascular segments; and
use the sum as a multivessel score that is a score element of the FFR impact score.

22. The apparatus of any one of claims 18-21, wherein the FFR impact score includes a sorted-value chart score element that represents the FFR values of each of the plurality of vascular segments in a combined value-sorted order, such that values from different vascular segments are interleaved with each other.

23. The apparatus of claim 22, wherein the processing device is configured to cause the user interface to display the sorted-value chart score element as a color-coded circle graph.

## Patentansprüche

1. Verfahren zum Schätzen eines klinischen Zustands eines Gefäßabschnitts, das von einer Verarbeitungsvorrichtung (608) durchgeführt wird, wobei das Verfahren umfasst:
Empfangen einer Karte einer fraktionellen Flussreserve (FFR), die mehrere FFR-Werte bestimmten Positionen auf jedem einer Mehrzahl von vaskulären Segmenten eines Gefäßbaums zuweist, der den Gefäßabschnitt darstellt;
Berechnen eines FFR-Auswirkungs-Scores unter Verwendung der abgebildeten FFR-Werte, wobei der FFR-Auswirkungs-Score eine gesamte Auswirkung einer obstruktiven Gefäßerkrankung auf den Gefäßbaum angibt;
wobei der FFR-Auswirkungs-Score ein kumulatives Diagramm umfasst und
wobei das Berechnen des FFR-Auswirkungs-Scores umfasst:
Entfernen der FFR-Werte von ihren abgebildeten Stellen; und
grafisches Darstellen der FFR-Werte in sortierter Reihenfolge,
wobei das grafische Darstellen der FFR-Werte weiter umfasst:
Umwandeln der FFR-Werte in das kumulative Diagramm.

2. Verfahren nach Anspruch 1, wobei die FFR-Karte fortlaufend oder nahezu fortlaufend FFR-Werte den bestimmten Positionen zuweist.

3. Verfahren nach einem der Ansprüche 1-2, wobei die FFR-Karte Beiträge zum Verringern einer Flusskapazität von einer stromaufwärts liegenden Position darstellt, die jeder der bestimmten Positionen gemein ist.

4. Verfahren nach einem der Ansprüche 1-3, wobei Berechnen des FFR-Auswirkungs-Scores Berechnen einer Mehrzahl von Score-Elementen umfasst, von welchen jedes Entfernen der Abbildung von FFR-Werten aus seiner abgebildeten Stelle umfasst, aber eine Zuordnung zu einem der Mehrzahl von Gefäßsegmenten beibehält.

5. Verfahren nach Anspruch 4, wobei die Mehrzahl von Score-Elementen einen Gesamtabnahme-Score für jedes der Mehrzahl von Gefäßsegmenten umfasst, der eine Gesamtabnahme in FFR entlang des Gefäßsegments von einem Referenzwert darstellt, der ein nicht okkludiertes Gefäßsystem darstellt.

6. Verfahren nach einem der Ansprüche 4-5, weiter umfassend Zuweisen zu jedem der Mehrzahl von Gefäßsegmenten eines Status als okkludiert oder nicht okkludiert basierend auf mindestens dem Gesamtabnahme-Score für das Gefäßsegment, Zählen einer Anzahl okkludierter Gefäßsegmente und Bereitstellen der Summe als einen Mehrfachgefäß-Score, der ein Score-Element des FFR-Auswirkungs-Scores ist.

7. Verfahren nach einem der Ansprüche 4-6, wobei die Mehrzahl von Score-Elementen einen maximalen Abnahme-Score für jedes der Mehrzahl von Gefäßsegmenten umfasst, der eine maximale Abnahme in FFR entlang des Gefäßsegments entlang eines definierten Abschnitts des Gefäßsegments darstellt, der kürzer als das gesamte Gefäßsegment ist.

8. Verfahren nach Anspruch 7, weiter umfassend Zuweisen zu jedem der Mehrzahl von Gefäßsegmenten eines Status als okkludiert oder nicht okkludiert basierend auf mindestens dem maximalen Abnahme-Score für das Gefäßsegment, Zählen der Anzahl okkludierter Gefäßsegmente und Bereitstellen der Summe als einen Mehrfachgefäß-Score, der ein Score-Element des FFR-Auswirkungs-Scores ist.

9. Verfahren nach einem der Ansprüche 4-8, wobei die Mehrzahl von Score-Elementen einen diffuse Score für jedes der Mehrzahl von Gefäßsegmenten umfasst, der ein Maß darstellt, wie weit verbreitet entlang eines Gefäßsegments Läsionen sind, die zu der Gesamtabnahme in FFR beitragen.

10. Verfahren nach Anspruch 9, wobei Berechnen des diffusen Gefäßabnahme-Scores umfasst:
Bestimmen eines Grads, bis zu dem sich der maximale Abnahme-Score von dem Gesamtabnahme-Score unterscheidet; oder
Berechnen eines Verhältnisses des Gesamtabnahme-Scores und des maximalen Abnahme-Scores.

11. Verfahren nach einem der Ansprüche 4-10, wobei die Mehrzahl von Score-Elementen umfasst:
einen Schweregrad-Score, der einen gewichteten Durchschnitt von FFR an allen Stellen in dem Gefäßbaum darstellt, der nach unten für Stellen mit zunehmend größerer Distanz von einem Ursprung des Gefäßbaums gewichtet ist; oder
ein oder mehrere Score-Elemente, die einem Segment zugeordnet sind, das eine linke Hauptkoronararterie bis zu einem ersten Verzweigungspunkt der linken Hauptkoronararterie umfasst, die in dem Gefäßbaum dargestellt ist.

12. Verfahren nach einem der Ansprüche 1-11, wobei der FFR-Auswirkungs-Score umfasst:
ein nach Wert sortiertes Diagramm-Score-Element, das die FFR-Werte jedes der Mehrzahl von Gefäßsegmenten in einer kombinierten, nach Wert sortierten Reihenfolge darstellt, sodass Werte von verschiedenen Gefäßsegmenten miteinander verschachtelt sind;
ein Histogramm-Score-Element, das die FFR-Werte jedes der Mehrzahl von Gefäßsegmenten in einem kombinierten Histogramm darstellt, wobei ein Beitrag jedes FFR-Werts zu dem Histogramm gemäß einer Magnitude eines Gefäßvolumens gewichtet ist, in dem der FFR-Wert auftritt; oder
ein Score-Element, das eine Läsionslänge basierend auf einer Distanz beschreibt, über die ein FFR-Wert fortlaufend abnimmt.

13. Verfahren nach Anspruch 12, weiter umfassend Anzeigen des nach Wert sortierten Diagramm-Score-Elements als ein farbcodiertes Kreisdiagramm.

14. Verfahren nach einem der Ansprüche 1-13, wobei der FFR-Auswirkungs-Score ein Score-Element umfasst, das eine Läsionsgeometrie als eines oder mehrere der Folgenden beschreibt:
sowohl ein Hauptgefäß als auch einen Seitenast enthaltend;
ein Hauptgefäß und mindestens zwei seiner Äste enthaltend;
in einem aorto-ostialen Gefäßsegment auftretend;
neben einem gewundenen Bereich des Gefäßsystems auftretend; oder
innerhalb eines gewundenen Bereichs des Gefäßsystems auftretend.

15. Verfahren nach einem der Ansprüche 1-14, weiter umfassend Einstellen des FFR-Auswirkungs-Scores basierend auf einer überarbeiteten FFR-Karte, die gemäß einem oder mehreren der Folgenden überarbeitet wurde:
automatisches virtuelles Stenting;
manuelle Zustandsauswahl;
Daten, die nach einer Stent-Implantation gemessen wurden; oder
Daten, die durch eine Mehrzahl von diagnostischen Prozeduren gemessen wurden.

16. Verfahren nach einem der Ansprüche 1-15, weiter umfassend Vergleichen des FFR-Auswirkungs-Scores mit einem zweiten FFR-Auswirkungs-Score, der nach dem Verfahren von Anspruch 1 berechnet wurde, und Schätzen einer Rate eines Fortschritts einer Gefäßerkrankung.

17. Verfahren nach Anspruch 16, weiter umfassend mindestens eines von:
Planen einer weiteren diagnostischen Prozedur basierend auf der Schätzung;
Planen einer Behandlungsprozedur basierend auf dem FFR-Auswirkungs-Score;
Auswählen zwischen einer OMT- und einer PCI-Behandlung basierend auf dem FFR-Auswirkungs-Score;
Auswählen zwischen einer PCI- und einer CABG-Behandlung basierend auf dem FFR-Auswirkungs-Score;
umfassend Planen mindestens eines von einer Anzahl, Stelle und Art von zu platzierenden Stents basierend auf dem FFR-Auswirkungs-Score; oder
umfassend Planen mindestens einer von einer Anzahl und/oder Stelle von zu platzierenden CABG-Transplantaten basierend auf dem FFR-Auswirkungs-Score.

18. Einrichtung (600) zum Schätzen eines klinischen Zustands eines Gefäßabschnitts, wobei die Einrichtung umfasst:
eine Datenspeichervorrichtung (604), die eine Datenstruktur speichert, die mehrere Werte einer fraktionellen Flussreserve (FFR) bestimmten Positionen auf jedem einer Mehrzahl von vaskulären Segmenten eines Gefäßbaums zuweist, der den Gefäßabschnitt darstellt; und
eine Verarbeitungsvorrichtung (608), die kommunikativ an die Datenspeichervorrichtung gekoppelt ist und eingerichtet ist zum:
Berechnen eines FFR-Auswirkungs-Scores unter Verwendung der zugewiesenen FFR-Werte, wobei die FFR-Auswirkungs-Scores eine gesamte Auswirkung einer obstruktiven Gefäßerkrankung auf den Gefäßbaum angeben;
wobei der FFR-Auswirkungs-Score ein kumulatives Diagramm umfasst,
und wobei das Berechnen des FFR-Auswirkungs-Scores umfasst:
Entfernen der FFR-Werte von ihren abgebildeten Stellen; und
grafisch Darstellen der FFR-Werte in sortierter Reihenfolge,
wobei das grafische Darstellen der FFR-Werte weiter umfasst:
Umwandeln der FFR-Werte in das kumulative Diagramm, und
Veranlassen, dass der FFR-Auswirkungs-Score auf einer Benutzeroberfläche (602) angezeigt wird.

19. Einrichtung nach Anspruch 18, wobei Berechnen des FFR-Auswirkungs-Scores Berechnen einer Mehrzahl von Score-Elementen umfasst, von welchen jedes Entfernen der Zuweisung von FFR-Werten aus seiner abgebildeten Stelle umfasst, aber eine Zuordnung zu einem der Mehrzahl von Gefäßsegmenten beibehält.

20. Einrichtung nach Anspruch 19, wobei die Mehrzahl von Score-Elementen einen Gesamtabnahme-Score für jedes der Mehrzahl von Gefäßsegmenten umfasst, der eine Gesamtabnahme in FFR entlang des Gefäßsegments von einem Referenzwert darstellt, der ein nicht okkludiertes Gefäßsystem darstellt.

21. Einrichtung nach einem der Ansprüche 19 oder 20, wobei die Verarbeitungsvorrichtung eingerichtet ist zum:
Zuweisen zu jedem der Mehrzahl von Gefäßsegmenten eines Status als okkludiert oder nicht okkludiert, basierend auf mindestens dem Gesamtabnahme-Score für das Gefäßsegment;
Zählen einer Anzahl okkludierter Gefäßsegmente; und
Verwenden der Summe als Mehrgefäß-Score, der ein Score-Element des FFR-Auswirkungs-Scores ist.

22. Einrichtung nach einem der Ansprüche 18-21, wobei der FFR-Auswirkungs-Score ein nach Wert sortiertes Diagramm-Score-Element enthält, das die FFR-Werte jedes der Mehrzahl von Gefäßsegmenten in einer kombinierten, nach Wert sortierten Reihenfolge darstellt, sodass Werte von verschiedenen Gefäßsegmenten miteinander verschachtelt sind.

23. Einrichtung nach Anspruch 22, wobei die Verarbeitungsvorrichtung eingerichtet ist zu veranlassen, dass die Benutzeroberfläche das nach Wert sortierte Diagramm-Score-Element als ein farbcodiertes Kreisdiagramm anzeigt.

## Revendications

1. Procédé d'estimation d'un état clinique d'une partie vasculaire exécuté par un dispositif de traitement (608), le procédé comprenant :
la réception d'une représentation de réserve de débit fractionnaire (FFR) qui attribue une multiplicité de valeurs de réserve de débit fractionnaire à des emplacements particuliers sur chacun d'une pluralité de segments vasculaires d'une arborescence vasculaire représentant la partie vasculaire ;
le calcul d'un score d'impact de réserve de débit fractionnaire à l'aide des valeurs de réserve de débit fractionnaire représentées, le score d'impact de réserve de débit fractionnaire indiquant un impact global d'une affection vasculaire occlusive sur l'arborescence vasculaire ;
dans lequel le score d'impact de réserve de débit fractionnaire comprend un graphique cumulatif, et
dans lequel le calcul du score d'impact de réserve de débit fractionnaire comprend :
la suppression des valeurs de réserve de débit fractionnaire de leurs emplacements représentés ; et
la représentation graphique des valeurs de réserve de débit fractionnaire dans un ordre trié,
dans lequel la représentation graphique des valeurs de réserve de débit fractionnaire comprend en outre :
la conversion des valeurs de réserve de débit fractionnaire en graphique cumulatif.

2. Procédé selon la revendication 1, dans lequel la représentation de la réserve de débit fractionnaire de manière continue ou quasi-continue attribue des valeurs de réserve de débit fractionnaire aux emplacements particuliers.

3. Procédé selon l'une quelconque des revendications 1 à 2, dans lequel le représentation de la réserve de débit fractionnaire représente les contributions à la réduction du pouvoir d'écoulement depuis un emplacement amont commun à chacun des emplacements particuliers.

4. Procédé selon l'une quelconque des revendications 1 à 3, dans lequel le calcul du score d'impact de réserve de débit fractionnaire comprend le calcul d'une pluralité d'éléments de score, dont chacun comprend la suppression de la représentation des valeurs de réserve de débit fractionnaire de son emplacement représenté, mais conserve une association avec l'un de la pluralité de segments vasculaires.

5. Procédé selon la revendication 4, dans lequel la pluralité d'éléments de score comprend un score de chute totale pour chacun de la pluralité de segments vasculaires, représentant une chute totale de la réserve de débit fractionnaire le long du segment vasculaire par rapport à une valeur de référence qui représente un système vasculaire non occlus.

6. Procédé selon l'une quelconque des revendications 4 à 5, comprenant en outre l'affectation, à chacun de la pluralité de segments vasculaires, d'un statut « occlus » ou « non occlus », sur la base au moins du score de chute totale pour le segment vasculaire, le décompte d'un nombre de segments vasculaires occlus, et la restitution de la somme sous la forme d'un score multi-vaisseaux qui est un élément de score du score d'impact de réserve de débit fractionnaire.

7. Procédé selon l'une quelconque des revendications 4 à 6, dans lequel la pluralité d'éléments de score comprend un score de chute maximale pour chacun de la pluralité de segments vasculaires, qui représente une chute maximale de la réserve de débit fractionnaire le long du segment vasculaire le long d'une partie définie du segment vasculaire plus courte que le segment vasculaire entier.

8. Procédé selon la revendication 7, comprenant en outre l'attribution, à chacun de la pluralité de segments vasculaires, d'un statut « occlus » ou « non occlus », sur la base au moins du score de chute maximale pour le segment vasculaire, le décompte du nombre de segments vasculaires occlus, et la restitution de la somme sous la forme d'un score multi-vaisseaux qui est un élément de score du score d'impact de réserve de débit fractionnaire.

9. Procédé selon l'une quelconque des revendications 4 à 8, dans lequel la pluralité d'éléments de score comprend un score diffus pour chacun de la pluralité de segments vasculaires, qui représente une mesure de l'étendue de la répartition, le long du segment vasculaire, des lésions qui contribuent à la chute totale de la réserve de débit fractionnaire.

10. Procédé selon la revendication 9, dans lequel le calcul du score diffus de chute vasculaire comprend :
la détermination d'un degré selon lequel le score de chute maximale diffère du score de chute totale ; ou
le calcul d'un rapport entre le score de chute totale et le score de chute maximale.

11. Procédé selon l'une quelconque des revendications 4 à 10, dans lequel la pluralité d'éléments de score comprend :
un score de gravité, qui représente une moyenne pondérée de la réserve de débit fractionnaire à tous les emplacements dans l'arborescence vasculaire, pondéré à la baisse pour les emplacements qui se trouvent à une distance de plus en plus importante d'une origine de l'arborescence vasculaire ; ou
un ou plusieurs éléments de score associés à un segment comprenant une artère coronaire principale gauche jusqu'à un premier point de ramification de l'artère coronaire principale gauche représentée dans l'arborescence vasculaire.

12. Procédé selon l'une quelconque des revendications 1 à 11, dans lequel le score d'impact de réserve de débit fractionnaire comprend :
un élément de score de graphique à valeurs triées qui représente les valeurs de réserve de débit fractionnaire de chacun de la pluralité de segments vasculaires dans un ordre de tri de valeurs combiné, de sorte que les valeurs de différents segments vasculaires sont entrelacées les unes avec les autres ;
un élément de score d'histogramme qui représente les valeurs de réserve de débit fractionnaire de chacun de la pluralité de segments vasculaires sur un histogramme combiné, dans lequel une contribution de chaque valeur de réserve de débit fractionnaire à l'histogramme est pondérée selon une ampleur du volume vasculaire dans lequel la valeur de réserve de débit fractionnaire se produit ; ou
un élément de score qui décrit une longueur de lésion sur la base d'une distance sur laquelle une valeur de réserve de débit fractionnaire diminue de manière continue.

13. Procédé selon la revendication 12, comprenant en outre l'affichage de l'élément de score de graphique à valeurs triées sous la forme d'un graphique circulaire à codage couleur.

14. Procédé selon l'une quelconque des revendications 1 à 13, dans lequel le score d'impact de réserve de débit fractionnaire comprend un élément de score qui décrit une géométrie de lésion comme un ou plusieurs de ce qui suit :
comme comprenant un vaisseau principal et une ramification latérale ;
comme comprenant un vaisseau principal et au moins deux de ses ramifications ;
comme se produisant dans un segment vasculaire aorto-ostial ;
comme se produisant de manière adjacente à une zone tortueuse du système vasculaire ; ou
comme se produisant dans une zone tortueuse du système vasculaire.

15. Procédé selon l'une quelconque des revendications 1 à 14, comprenant en outre l'ajustement du score d'impact de réserve de débit fractionnaire, sur la base d'une représentation révisée de la réserve de débit fractionnaire, révisée selon un ou plusieurs de ce qui suit :
une implantation d'endoprothèse vasculaire virtuelle automatique ;
une sélection d'état manuelle ;
des données mesurées après une implantation d'endoprothèse vasculaire ; ou
des données mesurées par le biais d'une pluralité de procédures de diagnostic.

16. Procédé selon l'une quelconque des revendications 1 à 15, comprenant en outre la comparaison du score d'impact de réserve de débit fractionnaire avec un deuxième score d'impact de réserve de débit fractionnaire calculé selon le procédé de la revendication 1, et l'estimation d'un taux de progression de l'affectation vasculaire.

17. Procédé selon la revendication 16, comprenant en outre au moins l'un de :
la planification d'une autre procédure de diagnostic, sur la base de l'estimation ;
la planification d'une procédure de traitement, sur la base du score d'impact de réserve de débit fractionnaire ;
le choix entre un traitement OMT et PCI, sur la base du score d'impact de réserve de débit fractionnaire ;
le choix entre un traitement PCI et CABG, sur la base du score d'impact de réserve de débit fractionnaire ;
comprenant la planification d'au moins l'un d'un nombre, d'un emplacement et/ou d'un type de prothèses endovasculaires à placer, sur la base du score d'impact de réserve de débit fractionnaire ; ou
comprenant la planification d'au moins l'un d'un nombre et/ou d'un emplacement, de greffes CABG à placer, sur la base du score d'impact de réserve de débit fractionnaire.

18. Appareil (600) destiné à estimer un état clinique d'une partie vasculaire, l'appareil comprenant :
un dispositif de stockage de données (604) qui stocke une structure de données qui attribue une multiplicité de valeurs de réserve de débit fractionnaire (FFR) à des emplacements particuliers sur chacun d'une pluralité de segments vasculaires d'une arborescence vasculaire représentant la partie vasculaire ; et
un dispositif de traitement (608) couplé en communication au dispositif de stockage de données et configuré pour :
calculer un score d'impact de réserve de débit fractionnaire à l'aide des valeurs de réserve de débit fractionnaire attribuées, les scores d'impact de réserve de débit fractionnaire indiquant un impact global d'une affection vasculaire occlusive sur l'arborescence vasculaire,
dans lequel le score d'impact de réserve de débit fractionnaire comprend un graphique cumulatif,
et dans lequel le calcul du score d'impact de réserve de débit fractionnaire comprend :
la suppression des valeurs de réserve de débit fractionnaire de leurs emplacements représentés ; et
la représentation graphique des valeurs de réserve de débit fractionnaire dans un ordre trié,
dans lequel la représentation graphique des valeurs de réserve de débit fractionnaire comprend en outre :
la conversion des valeurs de réserve de débit fractionnaire en graphique cumulatif, et
pour permettre à une interface utilisateur (602) d'afficher le score d'impact de réserve de débit fractionnaire.

19. Appareil selon la revendication 18, dans lequel le calcul du score d'impact de réserve de débit fractionnaire comprend le calcul d'une pluralité d'éléments de score, dont chacun comprend la suppression de l'attribution des valeurs de réserve de débit fractionnaire de son emplacement représenté, mais conserve une association avec l'un de la pluralité de segments vasculaires.

20. Appareil selon la revendication 19, dans lequel la pluralité d'éléments de score comprend un score de chute totale pour chacun de la pluralité de segments vasculaires, représentant une chute totale de la réserve de débit fractionnaire le long du segment vasculaire par rapport à une valeur de référence qui représente un système vasculaire non occlus.

21. Appareil selon l'une quelconque des revendications 19 ou 20, dans lequel le dispositif de traitement est configuré pour :
attribuer à chacun de la pluralité de segments vasculaires un statut « occlus » ou « non occlus », sur la base au moins du score de chute totale pour le segment vasculaire ;
décompter un nombre de segments vasculaires occlus ; et
utiliser la somme comme un score multi-vaisseaux qui est un élément de score du score d'impact de réserve de débit fractionnaire.

22. Appareil selon l'une quelconque des revendications 18 à 21, dans lequel le score d'impact de réserve de débit fractionnaire comprend un élément de score de graphique à valeurs triées qui représente les valeurs de réserve de débit fractionnaire de chacun de la pluralité de segments vasculaires dans un ordre de tri de valeurs combiné, de sorte que les valeurs de différents segments vasculaires sont entrelacées les unes avec les autres.

23. Appareil selon la revendication 22, dans lequel le dispositif de traitement est configuré pour permettre à l'interface utilisateur d'afficher l'élément de score de graphique à valeurs triées sous la forme d'un graphique circulaire à codage couleur.
